# EUROPEAN PATENT SPECIFICATION

(11) **EP 0 619 735 B1**
(45) Date of publication and mention of the grant of the patent: **24.05.2000**
(21) Application number: 92921457.5
(22) Date of filing: 22.10.1992
(51) Int. Cl.: A61K 31/495, A61K 9/14, C07D 241/08

(54) **LYOPHILIZED COMPOSITION CONTAINING S(+)-4,4'-(1-METHYL-1,2-ETHANEDIYL)-BIS(2,6-PIPERAZINEDIONE)**
LYOPHILISIERTE ZUSAMMENSETZUNG, DIE S(+)-4,4'-(1-METHYL-1,2-ETHANDIYL)-BIS(2,6-PIPERAZINDION)ENTHÄLT
COMPOSITION LYOPHILISEE CONTENANT S(+)-4,4'-(1-METHYL-1,2-ETHANEDIYL)-BIS(2,6-PIPERAZINEDIONE)

(30) Priority: 25.10.1991 GB 9122720
(43) Date of publication of application: 19.10.1994
(73) Proprietor: CHIRON CORPORATION, Emeryville California 94608-2916 (US)
(72) Inventor: Holthuis, Josephus Johannes Maria, 2334 AB Leiden (NL); Voetman, Alwinus Antonius, 1161 DI Zwanenburg (NL)
(74) Representative: Goldin, Douglas Michael
(86) International application number: EP9202400
(87) International publication number: WO9307873

(56) References cited:
- GB-A- 1 234 935
- US-A- 4 963 551

## Description

This invention relates to an improved process for preparing lyophilized (S) (+)-4,4'-(1-methyl-1,2-ethanediyl)-bis(2,6-piperazinedione) (hereinafter referred to as "ICRF-187") salts, and further provides new lyophilized preparations of these substances and their use. The invention is also concerned with new findings on light stability of ICRF-187 salts, and solutions thereof.

ICRF-187 was described by Creighton in, inter alia, US Patent 3,941,790 as a material useful for aiding regression and palliation of cancer in mammals. In Pathologie Biologie, 1987, 35 (No 1) 49-53, Green described how certain anthracyclines are effective anti-tumour agents but carry the side effect of a tendency to produce cardiotoxicity upon chronic administration. Green, however, also disclosed for the first time that ICRF-187 could protect against such cardiotoxicity. One particular widely used anti-cancer drug suffering from the disadvantage of associated cardiotoxicity is doxorubicin hydrochloride. In US Patent 4,963,551, Palepu et al described a method of formulating ICRF-187 in a manner stated as facilitating intravenous (I.V.) administration of this substance (and the manufacture of an I.V. product) as a cardioprotective agent to reduce or prevent cardiotoxicity resulting from the administration of doxorubicin hydrochloride.

As for the manufacture of ICRF-187, Creighton, supra, essentially described two methods. In the first method, 1,2-diaminopropane tetraacetic acid is heated with formamide to result in incorporation of nitrogen and ring closure. In the second method, the tetraamide corresponding to the above tetraacid is heated in polyphosphoric acid or phenol, bringing about cyclization.

US Patent 4,764,614 describes an alternative synthesis in which propylenediamine tetraacetic acid tetraamide is treated in a dipolar aprotic solvent with an alkali metal derivative of dimethyl sulfoxide to form a dialkali metal salt of the desired bis-piperazinedione, the desired heterocyclic product.

EP-A-O 330 381 describes yet an alternative process for preparing, inter alia, ICRF-187, in which a corresponding tetranitrile is synthesized by reacting an appropriate diamine with formaldehyde and an alkali metal cyanide. The tetranitrile is then hydrated to yield an acid addition salt of the corresponding tetraamide, and this latter substance may then be cyclized.

In Repta et al, J. Pharmaceutical Sciences, 65(No 2) 238-242, the tetraacid product is synthesized from 1,2-diaminopropane dihydrochloride by reaction with chloroacetic acid and sodium hydroxide, and removal of salt byproduct is achieved using a cation-exchange resin column operated at elevated temperature. This technique is, however, very cumbersome, and quite impractical for industrial scale operation.

Attempts at the separation of alkali metal salt and tetraacid product by fractional crystallization have resulted in significant product loss and consequent low overall yield of ICRF-187. Moreover, using alternative precipitation techniques for tetraacid isolation, an unprocessable gel often results from precipitating tetraacid product in the presence of alkali metal salt when using an organic solvent/water mix to achieve precipitation.

In a co-pending application filed in the names of EuroCetus BV and Sicor Limited (WO-A-9308172) a process is described for the preparation of ICRF-187 in which an intermediate 1,2-diaminopropane tetraacetic acid product is synthesized together with byproduct alkali metal salt, characterized in that the tetraacetic acid intermediate product is subjected to ring formation in the form of a crude product having substantial amounts of alkali metal salt thereby to produce the desired ICRF-187.

The formulations described in US Patent 4,963,551, supra, are stated to contain up to about 6% moisture, and yet be capable of being stored at room temperature. The specific embodiment described in US Patent 4,963,551 is of a lyophilized preparation in which the cake of lyophilizate appears pink and glossy with cracks. This cake is stated to contain about 6% moisture. It is acknowledged in US Patent 4,963,551, that the presence of more than 2% moisture in the lyophilizate will result in the formation of crystals. The prior art is, however, entirely silent on the effect of moisture on product decomposition and the effect of UV light on stability, and the effect of these influences in combination.

The lyophilization process of US Patent 4,963,551 has a relatively short primary drying time at a relatively low temperature. Including time under vacuum without heating, the primary drying stage in this prior art process lasts 23 hours, and the product temperature at the end of this stage is 10°C. We have now found that an appreciably higher drying temperature and/or a primary drying stage in which the time under heating is at least 30 hours (preferably 40 hours or more) and/or a secondary drying time in which the time under heating is no greater than 24 hours produces a product which contains appreciably less moisture, contains fewer colour-causing impurities and decomposition products, and which is therefore more acceptable clinically. The present invention is also based upon the discoveries that ICRF-187 salts are light sensitive, and are far more unstable in the presence of appreciable moisture content.

We have also found that a clinically satisfactory product, lacking appreciable evidence of coloured decomposition products, dictates a limit to the concentration of the bulk solution from which the present lyophilized preparation is made. US Patent 4,963,551 states that the preparation described therein is made from a bulk solution having a minimum content of ICRF-187 of 25 mg/ml, and even suggests an upper limit as high as 40 mg/ml. We have found that the production of a more satisfactory cake lacking the clinically unsatisfactory pink and glossy characteristics and cracking described in this prior art sets an upper limit of concentration on the bulk solution which is certainly no higher than 25 mg/ml, and preferably no higher than about 20 mg/ml.

Accordingly, in one aspect the present invention provides a process for preparing ICRF-187 comprising:
a) lyophilizing a bulk solution of ICRF-187 having a concentration of no more than about 25 mg/ml; and
b) ensuring that the lyophilized ICRF-187 contains less than or equal to 2% moisture, preferably no more than 1% moisture,
in which the primary drying stage of the lyophilization is at least about 30 hours in duration and/or the primary drying temperature is from 30 to 40°C, the overall drying time is at least 20 hours, the temperature rise during primary drying is sufficiently slow to avoid colouration and during at least part of the process precautions are taken to avoid

In a further aspect, the invention provides a stable lyophilized preparation of ICRF-187 in which the cake of ICRF-187 is protected against the deleterious effect of light, has a moisture content of not more than 2%, and is obtainable by the process of the invention.

Preferably the content of decomposition products A, B and C defined hereinafter in such a preparation is less than 3%, preferably less than 2%.

The invention further provides the use of such a preparation in the manufacture of a medicament for the treatment or prophylaxis of cancer or counteracting cardiotoxicity during the treatment of cancer.

The invention yet further provides a process for protecting ICRF-187 from degradation comprising:
a) lyophilizing an aqueous solution of ICRF-187 having a concentration of no more than about 25 mg/ml in which lyophilization the primary drying stage is at least about 30 hours in duration and/or the primary drying temperature is from 30 to 40°C; the overall drying time is at least 20 hours and the temperature rise during primary drying is sufficiently slow to avoid colouration;
b) ensuring that the lyophilized ICRF-187 contains less than or equal to 2% moisture; and
c) protecting the lyophilized ICRF-187 from light.

In the lyophilization process used in the present invention the primary drying stage lasts at least 30 hours, preferably at least 40 hours, and/or the primary drying temperature is from 30°C to 40°C. Preferably the secondary drying stage is less than 25 hours in duration and the secondary temperature is from 30°C to 40°C.

Our experiments have shown that ICRF-187 is very sensitive to decomposition in the presence of water.

During the sublimation period of the lyophilized process (primary drying), if the temperature is raised too fast colouration of the cake is observed (colouration of a white cake always points at decomposition). In addition, to this, a fast rise in temperature (during primary drying) can result in melting of the frozen ice and/or collapse of the cake structure, which results in a deformed cake and a high residual water content. We have found, however, that when a relatively long period of primary drying and in most cases secondary drying are followed, a white product and an intact cake is obtained.

Several other parameters during the freeze drying process are of importance. The freezing time is not, however, critical. The primary drying can generally start as soon as the product temperature is -50°C. The sublimination period is important as noted above. The secondary drying stage commences when the product temperature equals the plate temperature, and its length is controlled by the pressure rise step.

The primary drying stage generally lasts at least 30 hours, preferably 40 to 50 hours, but it may exceed 50 hours. For example, if the drying temperature is only about 10°C (as in the prior art process of US Patent 4963551), a primary drying time of 60 to 75 hours is appropriate. It will be appreciated that drying times during a lyophilization process are, in part, dictated by the size and nature of the equipment used. We have found that the number of vials employed, and altering the concentration of the starting ICRF-187 solution do not alter the timing. However, those of skill in the art will appreciate that increasing vial size and/or reducing the amount of solution in the vial prior to lyophilization reduces the required time period. Decreasing drying temperature, however, dictates a longer primary drying process in the present invention, and usually suggests a secondary drying stage of at least 10 hours, perhaps 15 to 25 hours. In US Patent 4,963,551, 100 cc vials were filled for the lyophilization of bulk solution, and a temperature of 10°C was used for primary drying. Nonetheless the primary drying stage lasted only 23 hours, and overall drying lasted only 43 hours including the secondary drying stage.

The following Table 1 sets out preferred parameters for the present process, and shows the alterations in those parameters which apply on changing vial size/arrangement or temperature. Given the relatively lengthy primary drying process envisaged in the present invention, it may transpire that no secondary drying period at all is required. In any event it is usually no greater than about 25 hours. Generally, it is also preferred to operate with a secondary drying temperature of from about 30°C to about 40°C. As an illustration of changes in secondary drying, operating under conditions where 100 ml vials loaded with half the original volume, the secondary drying period can be reduced to 80% of the original period, all other conditions remaining the same.

**Table 1**

| Operating conditions (b to f showing changes in a) | Freezing | Pr. Drying | Sec. Drying |
|---|---|---|---|
| a) Vial 36 ml 20 mg/ml N = 4000 | 1-2 hrs | 41-52 hrs | 16-25 hrs |
| Product end temp | -45°- -54°C | 32-38°C | 32-38°C |
| Shelf temp ramp | constant | 13°C/hr from -45° to +35°C | constant |
| b) Number of vials twice as at a) | No changes | (within limits in a) | (within limits in a) |
| c) conc. 25 mg/ml | No changes | (within limits in a) | (within limits in a) |
| d) Vial of 100 ml | Reduction of time by 70% | Reduction of time by 80% | Reduction of time by 50% |
| e) Same vial only 50% of fill volume of a) | Reduction of time by 50% | Reduction of time by 40% | Reduction of time by 20% |
| f) as a) except lower temperatures for drying stages | 1-2 hrs -45°- -54°C | Increase of time by 50% - 60-75 hrs at 10°C | Increase of of time by 15% - 19-28 hrs at 28°C |

It will be appreciated that in Table 1 above the timings and percentage increases/decreases are approximate and given for general guidance. In the present invention, even if overall a relatively high temperature of from about 30°C to about 40°C is maintained during both primary and secondary drying, it is unlikely that a satisfactory moisture level (2% or less) will be produced by overall drying of less than about 20 hours.

As a result of our experimentation, we can now report that lyophilized preparations in accordance with the present invention have a content of decomposition products, A, B and C (as hereinafter defined) less than or equal to 3%, preferably less than or equal to 2%. Such lyophilized preparations have been shown to be stable for at least two years at 25°, provided that the moisture content is no greater than 2%. A sterile cake of this type is white or off white in appearance, and in consequence far more acceptable to a clinician than the material of US Patent 4963551.

The following Table 2 provides a comparison of lyophilization protocols in accordance with the present invention and the prior art process of US Patent 4963551. We have found that only by following the principles set out above, of which the parameter ranges in Table 2 are ranges indicative of preferred embodiments of the invention, can obtain a white or off-white cake of acceptable appearance and highly desirable low moisture content. Moisture content much higher than 2% has a deleterious effect on stability (there being an increased tendency to produce decomposition products A, B and C and, as mentioned in US Patent 4963551, a tendency to crystallize), and the moisture content should ideally be less than 1%. To achieve this latter desideratum an overall drying period (assuming a temperature of from about 30°C to about 40°C for both drying stages) of about 65 hours to about 80 hours is required for a 36ml vial.

In Table 2 it can be seen that when the vial size is 36ml, the present process ideally requires a total drying time of over seventy hours at a 32°C to 38°C drying temperature. When the vial size is 100 ml (as with prior art), a high operating temperature is still necessary despite the reduction in drying time.

**Table 2**

| | Prior art 100 ml | Present invention 36 ml | Present invention 100 ml |
|---|---|---|---|
| Freezing | | | |
| Product temp (°C) | -38 | -45°- -54°C | -45°- -54°C |

| Primary drying | | | |
|---|---|---|---|
| Product temp at end of period (°C) | 10 | 32-38 | 32-38 |
| Duration of pr. dr. period (hrs) | 23 | 41-52 | 10-13 |

| Sec. drying | | | |
|---|---|---|---|
| Shelf temp ramp (°C/hr) | 1 | n/a temperature constant | n/a temperature constant |
| Duration temp ramp (hrs) | 20 | n/a temperature constant | n/a temperature constant |
| Production temp at end of sec. drying (°C) | 27 | 32-38 | 32-38 |
| Duration (hrs) | 20 | 16-25 | 8-12 |
| Drying time under vacuum (hrs) | 43 | 73-80 | 24-30 |

Table 3 below gives details of further preferred Examples of the present inventive process. These are not limiting on the invention, but are merely illustrative.

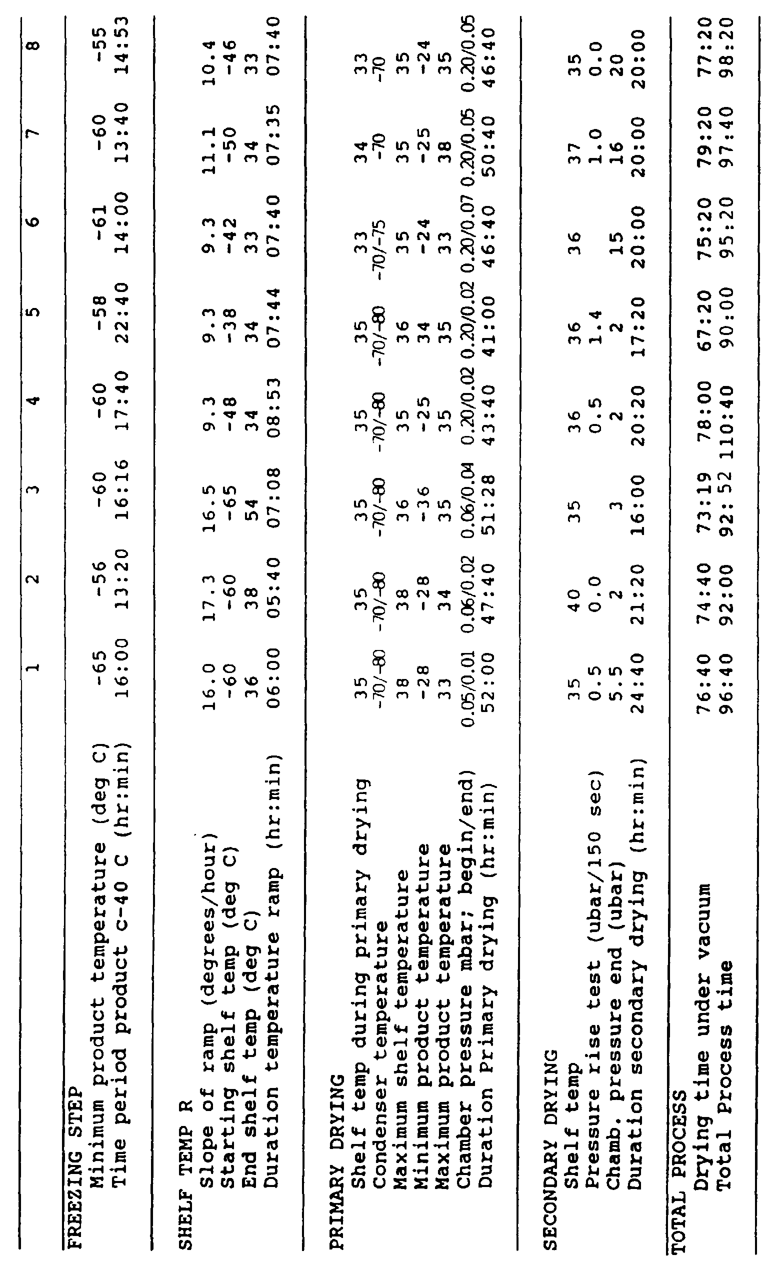

One very important advantage of the present invention is that accelerated stability studies at 60°C show that the present lyophilizated ICRF-187 preparations exhibit only a low degree of racemisation under such conditions.

In the present invention, it is preferred that the lyophilized preparation of an ICRF-187 salt is in the form of the hydrochloride salt. This can be achieved by freeze drying an HCl acid solution of ICRF-187.

A further aspect of the present invention is a process for preparation of a therapeutic formulation of a salt of ICRF-187 which comprises reconstituting a lyophilized preparation of the invention wherein the reconstituted therapeutic formulation has a degree of colouration no higher than Y4 (Eur.Ph.).

The present lyophilized products generally have a dissolution time of less than about 1 minute, usually less than about 0.5 minutes. When water is used for reconstitution purposes, the pH of a reconstituted hydrochloride product ranges from 1.4 to 1.8. Ideally the pH is about 1.5, at which pH about 90% of ICRF-187 is protonated. The resulting products are clear in appearance, and free of particulate matter.

A means for screening light may be used in protecting ICRF-187 from light-dependent decomposition. The screen can be any conventional screen known in the art, and generally is in the form of brown plastic or glass, eg a brown amber glass container meeting the specifications of 〈661〉 in USPXXII.

In general, drug clearance regulations do not accept the storage of new drugs in light resistant containers unless it has been shown that the drug in question is light sensitive. This is to ensure that clear vessels are generally employed, permitting visual inspection of product at all times. Hitherto, ICRF-187 has been stored in clear glass vessels without specific instructions for their storage in the dark. One extremely important aspect of the present invention is the finding of light sensitivity, and the requirement that this drug should now be stored in light resistant containers, such as brown glass vessels. Furthermore, it is now clearly desirable that operations employed in the preparation and handling of ICRF-187 preparations and formulations should take place against a background of light-reducing/eliminating precautions.

The importance of moisture is emphasised by our finding that lyophilized ICRF-187 degrades to the same extent over time at 4°C in the dark with 100% relative humidity as at 20°C under light conditions with 45% relative humidity. In one experiment, we found that a significant decrease in the weight percent of ICRF-187 was observed under both sets of conditions after 55 days. At 20°C, a combination of humidity and light may be responsible for this, and an increase in moisture content of the sample to 7.5% within 55 days was observed. Under the 4°C in the dark conditions only the high humidity can cause this degradation, and an increase in moisture content of the sample to 10.2% after 55 days of storage was seen. ICRF-187 clearly degrades in the dark very significantly under the influence of high humidity/moisture.

From the literature it is not known that ICRF-187 or salts, eg the hydrochloride salt, are light sensitive. The following Table 4 gives results of a controlled stability study we have performed showing that under the influence of light ICRF-187 hydrolyses more quickly than in the dark. After 57 days, although the content of ICRF-187 is still high, the colour of the cake and the colour of the reconstituted solution are unacceptable.

**Table 4**

| Time (days) | Total decomposition products % | | Decomposition products A+B % | |
|---|---|---|---|---|
| | Brown vial | Transparent vial | Brown vial | Transparent vial |
| 0 | 0.86 | 0.47 | 0.74 | 0.41 |
| 2 | 0.41 | 1.16 | 0.31 | 0.65 |
| 6 | 0.54 | 3.21 | 0.30 | 0.52 |
| 9 | 0.71 | 3.24 | 0.36 | 0.51 |
| 13 | 0.75 | 4.12 | 0.38 | 0.67 |
| 21 | 0.59 | 2.0 | 0.50 | 1.21 |
| 30 | 0.61 | 3.06 | 0.53 | 1.32 |
| 57 | 2.43 | 5.74 | 1.71 | 4.03 |
| | | | | |
| Slope | | | 0.0249 | 0.0607 |
| intercept | | | 0.0928 | 0.0607 |
| r | | | 0.9381 | 0.9518 |

The results presented in Table 5 below show that, in addition to the fast formation of A and B, a highly coloured decomposition product is also formed which results in a yellow coloured cake.

Decomposition products A, B and C referred to above are open-ring compounds of the following structures:-

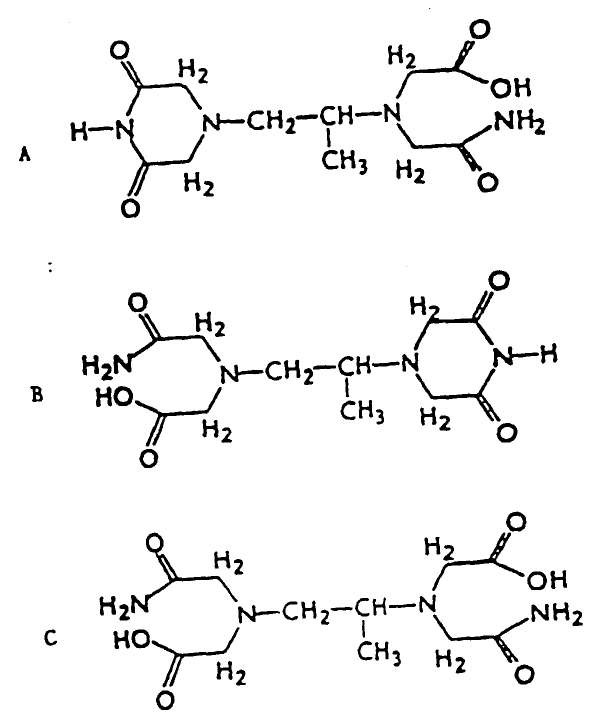

**Table 5**

| Time (days) | Appearance (colour cake) | | Colour of Solution | | Rate/extend of dissolution Clarity/Particles | | pm | |
|---|---|---|---|---|---|---|---|---|
| | 1 | 2 | 1 | 2 | 1 | 2 | 1 | 2 |
| 0 | white | white | B6 | Y6 | conform | conform | 1.60 | 1.64 |
| 2 | white | light yellow | Y5 | Y3 | conform | conform | 1.63 | 1.65 |
| 6 | white | yellow | Y6 | >Y1 | conform | conform | 1.60 | 1.61 |
| 9 | white | yellow | Y5 | >Y1 | conform | conform | 1.60 | 1.60 |
| 13 | white | yellow | Y5 | >Y1 | conform | conform | 1.60 | 1.60 |
| 21 | white | yellow | Y4 | >Y1 | conform | conform | 1.63 | 1.63 |
| 30 | white | yellow | Y4 | >Y1 | conform | conform | 1.52 | 1.61 |
| 57 | light yellow | yellow | Y2 | >Y1 | conform | conform | 1.63 | 1.62 |

The novel lyophilized preparations of ICRF-187 salts of the present invention can be formulated using any pharmaceutically acceptable acid to provide therapeutic formulations. Such acids include citric acid, phosphoric acid, hydrochloric acid and sulphuric acid. Dissolution time (based upon 20mg of product in 1.0ml of diluted acid) is preferably less than one minute, 20 seconds or so being desirable. Hydrochloric acid is the preferred acid because ICRF-187 is, we have found, soluble at an acceptable dissolution rate to produce a low pH solution (less than about 2.0). In contrast the rate of dissolution and the solubility of ICRF-187 in water or at pH 7.0 is limited, and stability at physiological pH is very limited.

The reconstituted formulations of the present invention are stable after reconstitution for at least 6 hours, which is quite adequate for clinical purposes. In fact at pH 1, ICRF-187 in solution seems to be stable for at least 2 days. At pH values higher than about 2, unstability of ICRF-187 solution progressively increases. We have found that reconstituted ICRF-187 formulations over longer periods of time show higher amounts of decomposition product A than of B and C regardless of light avoidance precautions. In general, light is a less important influence on stability for reconstituted formulations than the effect of the large amount of water present. This distinguishes from the relatively heightened importance of avoiding UV light in preserving lyophilized ICRF-187.

Other important aspects of the invention are:-
application for the treatment or prophylaxis of cancer, which involves administering an effective amount of the formulation of the invention; and
application for counteracting cardiotoxicity during drug therapy for the treatment or prophylaxis of cancer, which involves administering an effective amount of the formulation of the invention.

The present formulations are usable in clinical practice with dose levels of ICRF-187 of about 1000mg/m² per dose, in accordance with practice known in the art. The following outlines an acceptable procedure for making up material for clinical use. It should not be construed, however, as limiting the invention, merely showing a preferred embodiment.

### Example

A lyophilized product is first prepared as below, and with reference to Table 6.

**Table 6**

| Lyophilized Preparation | | | |
|---|---|---|---|
| Ingredients | Unit | Functions | Standard |
| Active ingredient | | | |
| ICRF-187 | 500 mg | Active ingredient | |

| Other constituents | | | |
|---|---|---|---|
| Concentrated hydrochloric acid (10 N) | 0.25 ml | To acidify the ICRF-187 solution | Ph.Eur. |
| Water for injection | 24.75 ml | To dilute the hydrochloric acid | Ph.Eur. |
| Nitrogen | | Vials are closed under a nitrogen atmosphere | N.F. / USP |

Each vial of lypholized ICRF-187 contains 500mg of ICRF-187 as active ingredient, formulated as hydrochloride salt. The vials are prepared by dissolving ICRF-187 in 0.1 N hydrochloric acid (at a concentration of 20 mg/ml of hydrochloric acid), sterilization of the fluid by filtration, filling of the vials with about 25 ml of the sterile solution, and lyophilization, in accordance with the inventive process as above.

Prior to use, the contents of the vials are dissolved in 25.0 ml of sterile water for injection (20 mg of ICRF-187 per ml), followed by neutralization with a specific volume of a sterile solution of 0.488 M sodium hydrogen phosphate in water for injection (pH 9.1; the volume to be used depends on the dose to be administered) and dilution with a sterile solution of 0.9 % sodium chloride in water for injection to a final volume of 200 ml, with a final pH of about 5.7. The final concentration in the infusion fluid is about 10 mg of ICRF-187 per ml.

The solution after reconstitution is acidic (pH 1.6), which results in higher stability. Because the compound has been shown to be sensitive to light, a brown glass, light-resistant vial is used.

In Table 7 below details are provided of the resulting product.

**Table 7**

| ICRF-187 infusion fluid | |
|---|---|
| The dose is calculated for a patient of 2.0 m² (1000 mg/m²) | |

| Product Characteristic | ICRF-187 |
|---|---|
| Dose | 2000 mg |
| Number of vials | 4 |

| Reconstitution | |
|---|---|
| solvent | water for injection |
| volume | 25.0 ml per vial |
| pH | 1.6 |
| concentration | 20 mg of ICRF-187 per ml |

| Dilution | |
|---|---|
| solvent | 0.448 M Na₂HPO₄ pH 9.1 |
| volume | 20.0 ml |
| solvent | water |
| volume | 80.0 ml |

| Final | |
|---|---|
| volume | 200 ml |
| concentration | 10 mg of ICRF-187 per ml |
| pH | 5.7 |

## Claims

1. A process for preparing a lyophilizate of (+)-4,4'-(1-methyl-1,2 ethanediyl)bis(2, 6-piperazinedione) (ICRF-187) comprising:
a) lyophilizing a bulk solution of ICRF-187 having a concentration of no more than about 25 mg/ml; and
b) ensuring that the lyophilized ICRF-187 contains less than or equal to 2% moisture,
in which the primary drying stage of the lyophilization is at least about 30 hours in duration and/or the primary drying temperature is from 30 to 40°C, the overall drying time is at least 20 hours, the temperature rise during primary drying is sufficiently slow to avoid colouration and during at least part of the process precautions are taken to avoid light.

2. A process as claimed in claim 1, wherein the moisture content of the lyophilized ICRF-187 is not more than about 1%.

3. A process as claimed in any preceding claim, wherein the ICRF-187 concentration of the bulk solution is no more than about 20 mg/ml.

4. A process as claimed in any preceding claim in which the primary drying stage is from 40 to 75 hours in duration and/or the secondary drying stage is less than 25 hours in duration.

5. A process as claimed in any preceding claim, wherein the secondary drying temperature is from 30°C to 40°C.

6. A process as claimed in any preceding claim, wherein the ICRF-187 is lyophilized as a therapeutically acceptable acid salt.

7. A process according to claim 6, wherein the therapeutically acceptable acid is citric acid, phosphoric acid, sulphuric acid or hydrochloric acid.

8. A process as claimed in any preceding claim, wherein the ICRF-187 is protected from light dependent decomposition using a light resistant container.

9. A process according to claim 8, wherein the light resistant container is a brown glass container.

10. A stable lyophilized preparation of ICRF-187 in which the cake of ICRF-187 is protected against the deleterious effect of light, has a moisture content of not more than 2%, and is obtainable by the process as claimed in any one of claims 1-9.

11. A preparation according to claim 10 having a content of the following decomposition products less than or equal to 3%:

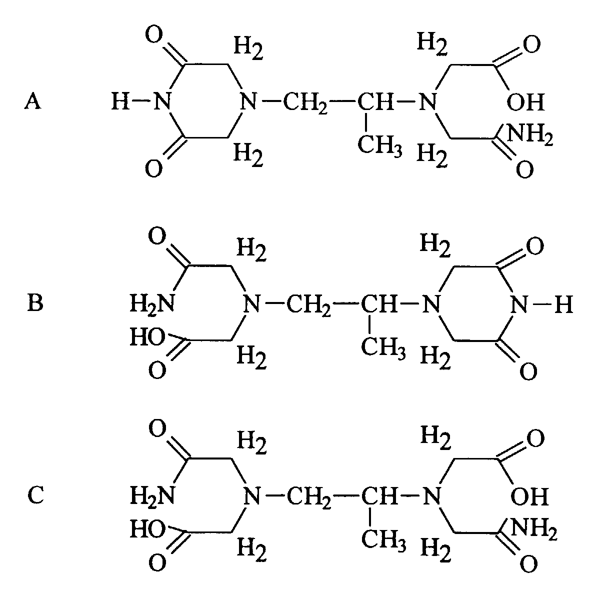

12. A process for preparation of a therapeutic formulation of ICRF-187, which comprises reconstituting a lyophilized preparation as defined in claim 10 or 11 wherein the reconstituted therapeutic formulation has a degree of colouration no higher than Y4 (Eur.Ph.).

13. A process according to claim 12, wherein the lyophilized ICRF-187 is in the form of a hydrochloride salt and the pH of the reconstituted formulation is from 1.4 to 1.8.

14. The use of a preparation as defined in claim 10 or 11 in the manufacture of a medicament for the treatment or prophylaxis of cancer or counteracting cardiotoxicity during the treatment of cancer.

15. A process for protecting (+)-4,4'-(1-methyl-1,2 ethanediyl)bis(2,6-piperazinedione) (ICRF-187) from degradation, comprising:
a) lyophilizing an aqueous solution of ICRF-187 having a concentration of no more than about 25 mg/ml in which lyophilization the primary drying stage is at least about 30 hours in duration and/or the primary drying temperature is from 30 to 40°C, the overall drying time is at least 20 hours, and the temperature rise during primary drying is sufficiently slow to avoid colouration;
b) ensuring that the lyophilized ICRF-187 contains less than or equal to 2% moisture; and
c) protecting the lyophilized ICRF-187 from light.

## Patentansprüche

1. Verfahren zur Herstelllung eines Lyophilisats von (+)-4,4'-(1-Methyl-1,2-ethandiyl)bis(2,6-piperazindion)(ICRF-187), umfassend:
a) Lyophilisieren einer Massenlösung von ICRF-187 mit einer Konzentration von nicht mehr als etwa 25 mg/ml; und
b) Sicherstellen, daß das lyophilisierte ICRF-187 weniger als oder gleich 2% Feuchtigkeit enthält,
wobei die primäre Trocknungsstufe der Lyophilisation mindestens etwa 30 Stunden dauert und/oder die primäre Trocknungstemperatur von 30 bis 40°C beträgt, die Gesamttrocknungszeit mindestens 20 Stunden beträgt, der Temperaturanstieg während des primären Trocknens genügend gering ist, um eine Färbung zu vermeiden und während mindestens eines Teils des Verfahrens Vorkehrungen getroffen werden, um Licht zu vermeiden.

2. Verfahren nach Anspruch 1, wobei der Feuchtigkeitsgehalt des lyophilisierten ICRF-187 nicht mehr als etwa 1% beträgt.

3. Verfahren nach einem der vorstehenden Ansprüche, wobei die ICRF-187-Konzentration der Massenlösung nicht mehr als etwa 20 mg/ml beträgt.

4. Verfahren nach einem der vorstehenden Ansprüche, bei dem die primäre Trocknungsstufe 40 bis 75 Stunden dauert und/oder die sekundäre Trocknungsstufe weniger als 25 Stunden dauert.

5. Verfahren nach einem der vorstehenden Ansprüche, wobei die sekundäre Trocknungstemperatur von 30°C bis 40°C beträgt.

6. Verfahren nach einem der vorstehenden Ansprüche, wobei das ICRF-187 als therapeutisch annehmbares Säuresalz lyophilisiert wird.

7. Verfahren nach Anspruch 6, wobei die therapeutisch annehmbare Säure Zitronensäure, Phosphorsäure, Schwefelsäure oder Salzsäure ist.

8. Verfahren nach einem der vorstehenden Ansprüche, wobei das ICRF-187 vor lichtabhängiger Zersetzung durch Verwendung eines lichtdichten Behälters geschützt wird.

9. Verfahren nach Anspruch 8, wobei der lichtdichte Behälter ein brauner Glasbehälter ist.

10. Ein stabiles lyophilisiertes Präparat von ICRF-187, bei dem der ICRF-187-Kuchen gegen den schädlichen Einfluß von Licht geschützt ist, einen Feuchtigkeitsgehalt von nicht mehr als 2% aufweist und durch ein Verfahren nach einem der Ansprüche 1 bis 9 erhalten werden kann.

11. Verfahren nach Anspruch 10 mit einem Anteil an folgenden Zersetzungsprodukten geringer oder gleich 3%:

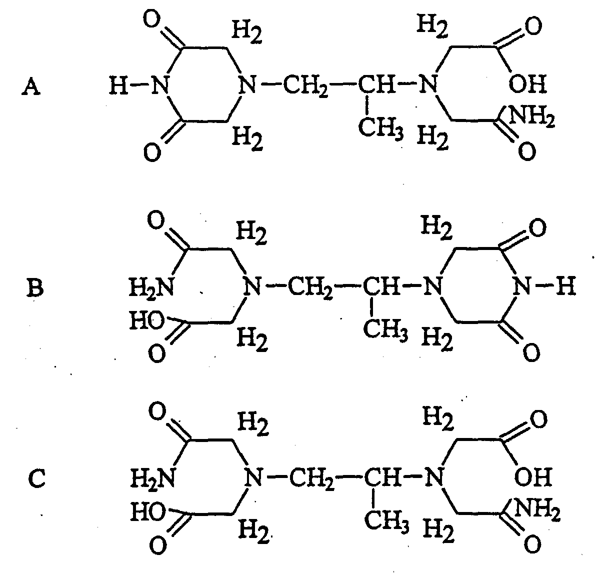

12. Verfahren zur Herstellung einer therapeutischen Formulierung von ICRF-187, welches es umfaßt, ein lyophilisiertes Präparat nach Anspruch 10 oder 11 wieder herzustellen, wobei die wieder hergestellte therapeutische Formulierung einen Färbungsgrad von nicht mehr als Y4 (Eur.Ph.) aufweist.

13. Verfahren nach Anspruch 12, wobei das lyophilisierte ICRF-187 in Form eines Hydrochloridsalzes vorliegt und der pH der wieder hergestellten Formulierung von 1,4 bis 1,8 beträgt.

14. Verwendung eines Präparats nach Anspruch 10 oder 11 zur Herstellung eines Medikaments für die Behandlung oder Prophylaxe von Krebs oder um der Kardiotoxizität während der Behandlung von Krebs entgegenzuwirken.

15. Verfahren zum Schützen von (+)-4,4'-(1-Methyl-1,2-ethandiyl)bis(2,6-piperazindion)(ICRF-187) vor Abbau, umfassend:
a) Lyophilisieren einer wäßrigen Lösung von ICRF-187 mit einer Konzentration von nicht mehr als etwa 25 mg/ml, wobei während dieser Lyophilisation die primäre Trocknungsstufe mindestens etwa 30 Stunden dauert und/oder die primäre Trocknungstemperatur von 30 bis 40°C beträgt, die Gesamttrocknungszeit mindestens 20 Stunden ist, und der Temperaturanstieg während des primären Trocknens genügend langsam ist, um eine Färbung zu vermeiden;
b) Sicherstellen, daß das lyophilisierte ICRF-187 weniger oder gleich 2% Feuchtigkeit enthält;
c) Schützen des lyophilisierten ICRF-187 vor Licht.

## Revendications

1. Procédé de préparation d'un lyophilisat de (+)-4,4'-(1-méthyl-1,2-éthanediyl)bis(2,6-pipérazinedione) (ICRF-187) comprenant les étapes consistant :
a) à lyophiliser un grand volume de solution de ICRF-187 ayant une concentration inférieure ou égale à environ 25 mg/ml ; et
b) à s'assurer que la ICRF-187 lyophilisée a une teneur en humidité inférieure ou égale à 2%,
dans lequel la durée de l'étape de séchage primaire de la lyophilisation est d'au moins 30 heures et/ou la température de séchage primaire va de 30 à 40°C, la durée globale du séchage est d'au moins 20 heures, l'augmentation de température pendant le séchage primaire est suffisamment lente pour éviter une coloration, et pendant au moins une partie du procédé, des précautions sont prises pour éviter la lumière.

2. Procédé selon la revendication 1, dans lequel la teneur en humidité de la ICRF-187 lyophilisée n'est pas supérieure à environ 1%.

3. Procédé selon l'une quelconque des revendications précédentes, dans lequel la concentration de ICRF-187 dans le grand volume de solution n'est pas supérieure à environ 20 mg/ml.

4. Procédé selon l'une quelconque des revendications précédentes, dans lequel la durée de l'étape primaire de séchage va de 40 à 75 heures et/ou la durée de l'étape de séchage secondaire est inférieure à 25 heures.

5. Procédé selon l'une quelconque des revendications précédentes, dans lequel la température de l'étape de séchage secondaire est de 30°C à 40°C.

6. Procédé selon l'une quelconque des revendications précédentes, dans lequel la ICRF-187 est lyophilisée sous forme de sel d'acide thérapeutiquement acceptable.

7. Procédé selon la revendication 6, dans lequel l'acide thérapeutiquement acceptable est l'acide citrique, l'acide phosphorique, l'acide sulfurique ou l'acide chlorhydrique.

8. Procédé selon l'une quelconque des revendications précédentes, dans lequel la ICRF-187 est protégée contre une décomposition dépendant de la lumière en utilisant un récipient résistant à la lumière.

9. Procédé selon la revendication 8, dans lequel le récipient résistant à la lumière est un récipient en verre brun.

10. Préparation lyophilisée stable de ICRF-187, dans laquelle le gâteau de ICRF-187 est protégé de l'effet nuisible de la lumière, a une teneur en humidité non supérieure à 2%, et peut être obtenu par le procédé selon l'une quelconque des revendications 1-9.

11. Préparation selon la revendication 10, ayant une teneur inférieure ou égale à 3% en produits de décomposition suivants:

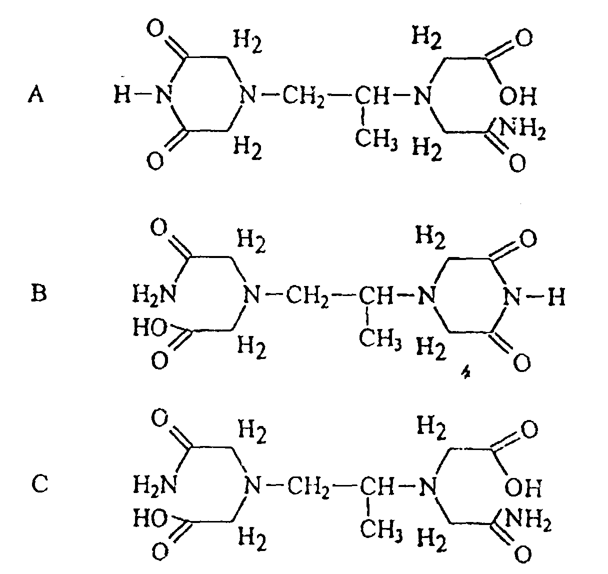

12. Procédé de préparation d'une formulation thérapeutique de ICRF-187, qui comprend la reconstitution d'une préparation lyophilisée définie dans la revendication 10 ou 11, la formulation thérapeutique reconstituée ayant un degré de coloration non supérieur à Y4 (Eur. Ph.).

13. Procédé selon la revendication 12, dans lequel la ICRF-187 lyophilisée est sous forme d'un chlorhydrate et le pH de la formulation reconstituée est de 1,4 à 1,8.

14. Utilisation d'une préparation telle que définie dans la revendication 10 ou 11 dans la fabrication d'un médicament destiné au traitement ou à la prophylaxie du cancer ou destiné à s'opposer à la cardiotoxicité au cours du traitement contre le cancer.

15. Procédé de protection de la (+)-4,4'-(1-méthyl-1,2-éthanediyl)bis(2,6-pipérazinedione) (ICRF-187) contre la dégradation, comprenant les étapes consistant :
a) à lyophiliser une solution aqueuse de ICRF-187 ayant une concentration non supérieure à environ 25 mg/ml, lyophilisation dans laquelle l'étape de séchage primaire de la lyophilisation a une durée d'au moins 30 heures et/ou la température de séchage primaire est de 30 à 40°C, la durée globale du séchage est d'au moins 20 heures, et l'augmentation de température pendant le séchage primaire est suffisamment lente pour éviter une coloration ;
b) à s'assurer que la ICRF-187 lyophilisée a une teneur en humidité inférieure ou égale à 2% ; et
c) à protéger la ICRF-187 lyophilisée de la lumière.
